# EUROPEAN PATENT SPECIFICATION

(11) **EP 1 107 787 B1**
(45) Date of publication and mention of the grant of the patent: **14.05.2003**
(21) Application number: 99944497.9
(22) Date of filing: 24.08.1999
(51) Int. Cl.: A61K 39/295, A61K 39/112, C12P 19/04, A61P 31/04, A61P 31/14

(54) **SALMONELLA TYPHI VACCINE COMPOSITIONS**
SALMONELLA TYPHI IMPFSTOFFZUSAMMENSETZUNGEN
COMPOSITIONS VACCINALES CONTENANT SALMONELLA TYPHI

(30) Priority: 28.08.1998 GB 9818910; 20.04.1999 GB 9909080
(43) Date of publication of application: 20.06.2001
(60) Divisional application: 02079708.0
(73) Proprietor: GlaxoSmithKline Biologicals S.A., 1330 Rixensart (BE)
(72) Inventor: DEMIL, Pascale, SmithKline Beecham, B-1330 Rixensart (BE); D'HONDT, Erik, SmithKline Beecham, B-1330 Rixensart (BE); VAN HOECKE, Christian, SmithKline Beecham, B-1330 Rixensart (BE)
(74) Representative: Privett, Kathryn Louise
(86) International application number: EP9906202
(87) International publication number: WO00012129

(56) References cited:
- US-A- 5 204 098
- VAN HOECKE C ET AL: "Concomitant vaccination against hepatitis A and typhoid fever." J TRAVEL MED, (1998 SEP) 5 (3) 116-20. , XP000891241
- DATABASE WPI Section Ch, Week 199850 Derwent Publications Ltd., London, GB; Class B04, AN 1998-592747 XP002133680 & RU 2 111 012 C (LVOV V L), 20 May 1998 (1998-05-20)

## Description

This invention relates to novel vaccine formulations, methods for preparing them and their use in therapy. In particular the present invention relates to combination vaccines for administration to travellers.

Typhoid fever is an acute generalised infection caused by *Salmonella typhi*, an organism for which humans are the only reservoir. The disease affects the reticuloendothelial system, intestinal lymphoid tissue and gall bladder. The case fatality rate in untreated patients suffering from severe typhoid fever is 9 to 32%. The risk of typhoid fever for travellers varies in relation to the incidence in the countries visited. For American travellers, the risk of typhoid infection is more than 10 per 100,000 if the destination is the Indian subcontinent. A study from New York City showed that of 479 cases of typhoid fever reported, 67% were travel-related and the mortality rate was 1.5%.

The vaccine Typherix (Trade Mark) which is a Vi polysaccharide typhoid vaccine may be used to protect against typhoid. Typherix™ is presented in pre-filled syringes, and contains 25 µg of Vi antigen per 0.5 ml dose. The conventional method of preparing the Vi polysaccharide typhoid vaccine involves phenol in order to stabilise the polysaccharide. Any combination vaccines prepared with the Vi polysaccharide typhoid vaccine have until now resulted in the other antigens present in the combination being unstable as a result of the presence of the phenol.

It has now been surprisingly found that vaccines comprising Typherix in combination with other antigens such as hepatitis A, hepatits B, Dengue and hepatitis E antigens are stable if the vaccine is formulated in a specific manner.

Certain parties are at an increased risk of becoming infected with typhoid, hepatitis A or hepatitis B . It is important to be protected effectively as soon as possible and in a simple way, most preferably in one dose. Examples of such parties include; clinical departments for tropical and infectious diseases; medical units caring for immuno-compromised patients; laboratories; development aid volunteers and their families; peace corps or militaries or persons acting in endemic areas.

A further important group of people for which accelerated vaccination is crucial is that of travellers. Coming from non-endemic areas, most travellers are not protected from infectious diseases. In Germany for instance, less than 20 % of the population aged 40 years or younger are seronegative against hepatitis A. Two thirds of the calculated 50,000 infections per year are imported by travellers. Preferred destinations of tourism like the tropics in Africa or South-East Asia are endemic for hepatitis B. Supported by WHO funded world-wide vaccination campaigns against hepatitis B in infants and children, an increasing number of tourists are aware of the potential risk and request also to be vaccinated against hepatitis B. However, the critical problem in most cases is the limited time frame of usually less than 4 weeks before departure.

Thus for these groups of people there is a need for a single vaccination for protection against typhoid and other diseases that travellers are prone to catch.

Van Hocke et al (J. Travel Med. 1998; 5:116 - 120) disclose results of a clinical trial to evaluate reactogenicty and immunogenicity of a Hepatitis A vaccine administered simultaneously with a Vi polysaccharide typhoid vaccine.

US 5,204,098 discloses that Vi capsular polysaccharides conjugated to toxin-dependent proteins can be used to enhance antibody response and to convert T-dependent properties to the Vi capsular polysaccharide.

Derwent publication XP-002133680 discloses a purification method for Vi antigen, the antigen then being used in a vaccine preparation with phenol.

WO9211291 discloses antigens comprising a hybrid polypeptide of Hepatitis B surface antigen and a heterologous antigen.

The present invention provides a vaccine composition comprising:
(a) a *Salmonella typhi* purified Vi polysaccharide,
(b) a hepatitis A antigen; and
(c) an aluminium salt
wherein the purification of the Vi polysaccharide does not contain phenol, such that:
when the Vi polysaccharide is combined with a hepatitis A antigen in solution the Vi retains stability and the hepatitis A antigen is not affected by the detrimental effects of phenol and the vaccine components are stable and do not interfere with each other.

The compositions of the invention may additionally comprise an adjuvant, more preferably a preferential stimulator of TH1 cell response.

It has been found that the vaccine compositions according to the invention surprisingly show no interference, that is to say that the immune response to each antigen in the composition of the invention is essentially the same as that which is obtained by each antigen given individually in conjunction with an adjuvant. The purification of the Vi polysaccharide does not contain phenol but instead the polysaccharide is stabilised by dehydrating with buffer in the absence of phenol.

Surprisingly, when the Vi polysaccharide is combined with another antigen, preferably Hepatitis A such as in the commercial vaccine HAVRIX™, in solution, the Vi retains stability and the other antigen(s) are not affected by the possible detrimental effects of phenol.

In a further aspect, the invention provides a vaccine composition comprising:
(a) a *Salmonella typhi* purified Vi polysaccharide and
(b) an hepatitis A (HAV) antigen
wherein the vaccine components are stable and do not interfere with each other.

Hepatitis A, caused by the *hepatitis A* virus, has a faecal-oral route of transmission and is associated with low levels of hygiene and overcrowding. Infection results in symptoms ranging from fever, anorexia, fatigue, nausea and vomiting to jaundice. About 1.4 million cases occur worldwide each year, but case fatality is low and age-specific with more deaths occurring in adults than in children. The disease is self-limiting and debilitating with no known effective treatment. Only short-term (4-6 months) passive prevention was available through the use of immunoglobulins, until the licensure of the first safe and immunogenic inactivated hepatitis A vaccine (Havrix™) in the early 1990's.

Vaccines for the prophylaxis of hepatitis A are now well known. The vaccine Havrix (Trade Mark), from SmithKline Beecham Biologicals can be used to prevent hepatitis A infections and is formulated with aluminium hydroxide as adjuvant. This vaccine comprises an attenuated strain of the HM-175 Hepatitis A virus inactivated with formol (formaldehyde); see Andre et al [Prog Med. Virol. 1990, vol 37; p72-95].

The formalin-inactivated hepatitis A monovalent vaccine in adults, Havrix™ 1440, contains at least 1440 EL.U of hepatitis A antigen per I ml dose. Extensive use of the vaccine in clinical trials and through commercial distribution has confirmed its safe, clinically well-tolerated, and highly immunogenic profile.

The hepatitis A antigen is preferably the HM-175 strain used in the commercial product Havrix (SmithKline Beecham Biologicals).

The concentration of hepatitis A antigen in the vaccine formulation of the invention is preferably about 720-2880 EU units per ml. For the definition of EU units see Andre et al (1990) loc cit.

The compositions of the invention which comprise HAV may additionally comprise aluminium hydroxide, the total amount of aluminium hydroxide generally being 0.05-0.10 mg per ml.

The total amount of aluminium salt per 0.5 or 1 ml dose is normally in the range 0.4-1.0mg.

In the vaccine composition of the invention it is advantageous to add formol (formaldehyde) such that the formol concentration is 10-200ug per ml.

Preferably the formol concentration is about 20-160 ug per ml.

With the overlap in countries where hepatitis A and typhoid fever are endemic, the opportunity to be vaccinated against two diseases in one shot will be attractive for business travellers and tourists to such regions. The convenience of one combined vaccine against both diseases will increase compliance. Thus the vaccine composition of the invention is of great benefit for administration to travellers who may be particularly at risk of typhoid and and/or hepatitis A infection .

Optionally the vaccine composition of the invention additionally comprises one or more of a number of other antigens, such as hepatitis B, dengue or hepatitis E.

Preferred dengue antigens include the envelope (E) glycoprotein proteins, among them truncated (at the carboxy-terminus) E proteins ( for example 60% E, 80% E or the B domain which is amino acids 301-395, or other fusions/portions thereof). For a reference see WO 96/37221. Other preferred dengue antigens include dengue viral proteins (E) deleted at their Carboxy-terminus and then fused to a Histidine-tail for example (WO 97/18311).

Preferred Hepatitis E antigens include Sar 55 available from Dyncorp and expressed in Baculovirus.

Vaccines for the prophylaxis of hepatitis B infections, comprising one or more hepatitis B antigens, are also well known. For example the vaccine Engerix-B (Trade Mark) from SmithKline Beecham Biologicals is used to prevent Hepatitis B. This vaccine comprises hepatitis B surface antigen (specifically the 226 amino acid S- antigen described in Harford et. al. in Postgraduate Medical Journal, 1987, 63 (Suppl. 2), p65-70) and is formulated using aluminium hydroxide as adjuvant.

Normally the hepatitis B antigen will be hepatitis B surface antigen (HBsAg). The preparation of Hepatitis B surface antigen (HBsAg) is well documented. See for example, Harford et al in Develop. Biol. Standard 54, page 125 (1983), Gregg et al in Biotechnology, 5, page 479 (1987), EP-A- 0 226 846, EP-A-0 299 108 and references therein.

As used herein the expression 'Hepatitis B surface antigen' or 'HBsAg' includes any HBsAg antigen or fragment thereof displaying the antigenicity of HBV surface antigen. It will be understood that in addition to the 226 amino acid sequence of the HBsAg S antigen (see Tiollais et al, Nature, 317, 489 (1985) and references therein) HBsAg as herein described may, if desired, contain all or part of a pre-S sequence as described in the above references and in EP-A- 0 278 940. HBsAg as herein described can also refer to variants, for example the 'escape mutant' described in WO 91/14703. In a further aspect the HBsAg may comprise a protein described as SL* in European Patent Application Number 0 414 374, that is to say a protein, the amino acid sequence of which consists of parts of the amino acid sequence of the hepatitis B virus large (L) protein (ad or ay subtype), characterised in that the amino acid sequence of the protein consists of either:
(a) residues 12 - 52, followed by residues 133 - 145, followed by residues 175 - 400 of the said L protein; or
(b) residue 12, followed by residues 14 - 52, followed by residues 133 - 145, followed by residues 175 - 400 of the said L protein.
HBsAg may also refer to polypeptides described in EP 0 198 474 or EP 0 304 578.

Normally the HBsAg will be in particle form. It may comprise S protein alone or may be as composite particles, for example (L*,S) wherein L* is as defined above and S denotes the S-protein of hepatitis B surface antigen.

The concentration of hepatitis B antigen in the vaccine formulation of the invention is preferably about 5 - 30µg per dose.

Preferably the HBsAg will be adsorbed on aluminium phosphate as described in WO93/24148.

Preferably the hepatitis B antigen is HBsAg S-antigen as used in the commercial product Engerix-B (Trade Mark).

The vaccine formulations of the present invention will contain an immunoprotective quantity of the antigens and may be prepared by conventional techniques. Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The vaccine compositions of the invention are preferably administered in one dose.

The vaccine compositions of the present invention are especially appropriate for adults and are also appropriate for administration to adolescents.

Adjuvants which are capable of preferential stimulation of the TH1 cell response are described in International Patent Application No. WO 94/00153 and WO 95/17209.

3 De-O-acylated monophosphoryl lipid A (3D-MPL) is one such adjuvant. This is known from GB 2220211 (Ribi). Chemically it is a mixture of 3 De-O-acylated monophosphoryl lipid A with 4, 5 or 6 acylated chains and is manufactured by Ribi Immunochem Montana. A preferred form of 3 De-O-acylated monophosphoryl lipid A is disclosed in EP0689454B 1 in the name of SmithKline Beecham Biologicals SA.

Preferably, the size of the particles of 3D-MPL is no greater than 120nm, normally 60-120nm, preferably about or less than 100nM (as described in European Patent number 0689454).

3D-MPL will be present in the range of 10µg - 100µg preferably 25-50µg per dose wherein the antigen will typically be present in a range 2-50µg per dose.

Another preferred adjuvant comprises QS21, an HPLC purified non-toxic fraction of a saponin from the bark of the South American tree Quillaja Saponaria Molina. m Optionally this may be admixed with 3 De-O-acylated monophosphoryl lipid A (3D-MPL), optionally together with an carrier.

The method of production of QS21 is disclosed (as QS21) in US patent No. 5,057,540.

Non-reactogenic adjuvant formulations containing QS21 have been described previously (WO 96/33739). Such formulations comprising QS21 and cholesterol have been shown to be successful TH 1 stimulating adjuvants when formulated together with an antigen. Thus vaccine compositions which form part of the present invention may include a combination of QS21 and cholesterol.

Combinations of different TH1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of 1 : 10 to 10 : 1; preferably 1:5 to 5 : 1 and often substantially 1 : 1. The preferred range for optimal synergy is 2.5 : 1 to 1 : 1 3D-MPL: QS21.

Preferably a carrier is also present in the vaccine composition according to the invention. The carrier may be an oil in water emulsion, or an aluminium salt.

A preferred oil-in-water emulsion comprises a metabolisible oil, such as squalene, alpha tocopherol and tween 80. Additionally the oil in water emulsion may contain span 85 and/or lecithin.

In a preferred aspect aluminium hydroxide (alum) or aluminium phosphate will be added to the composition of the invention to enhance immunogenicity.

In another preferred aspect the antigens in the vaccine composition according to the invention are combined with 3D-MPL and alum.

Typically for human administration QS21 and 3D-MPL will be present in a vaccine in the range of 1 µg - 200 µg, such as 10-100 µg, preferably 10 µg - 50 µg per dose. Typically the oil in water will comprise from 2 to 10% squalene, from 2 to 10% alpha tocopherol and from 0.3 to 3% tween 80. Preferably the ratio of squalene: alpha tocopherol is equal or less than 1 as this provides amore stable emulsion. Span 85 may also be present at a level of 1%. In some cases it may be advantageous that the vaccines of the present invention will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

They provide excellent protection against primary infection and stimulate, advantageously both specific humoral (neutralising antibodies) and also effector cell mediated (DTH) immune responses.

In a further aspect of the present invention there is provided a method of manufacture as herein described, wherein the method comprises preparation of the Vi polysaccharide in the absence of phenol making it both stable and suitable for making a combination vaccine.

The amount of protein in each vaccine dose is selected as an amount which induces an immunoprotective response without significant, adverse side effects in typical vaccinees. Such amount will vary depending upon which specific immunogen is employed. Generally, it is expected that each dose will comprise 1-1000 µg of protein, preferably 2-100 µg, most preferably 4-40 µg. An optimal amount for a particular vaccine can be ascertained by standard studies involving observation of antibody titres and other responses in subjects.

In addition to vaccination of persons susceptible to Typhoid fever or HAV infections, the pharmaceutical compositions of the present invention may be used to treat, immunotherapeutically, patients suffering from the infections.

The following examples illustrate the invention.

### Examples

### Example 1: Production of Vi polysaccharide

### Manufacture of the Vi polysaccharide

Essentially, the Vi polysaccharide production procedure involves the following steps :
fermentation of *Salmonella typhi* bacteria
extraction/purification of the polysaccharide

The fermentation is based on the seed lot principle. Each production run is initiated from one vial of *Salmonella typhi* working seed lot.

The production of the working seed followed by the description of the different steps of Vi polysaccharide production is given hereafter.

### Production of the working seed

A summary of the manufacturing steps and QC testing is shown in Scheme 1 below.
A description of each step is given hereafter.

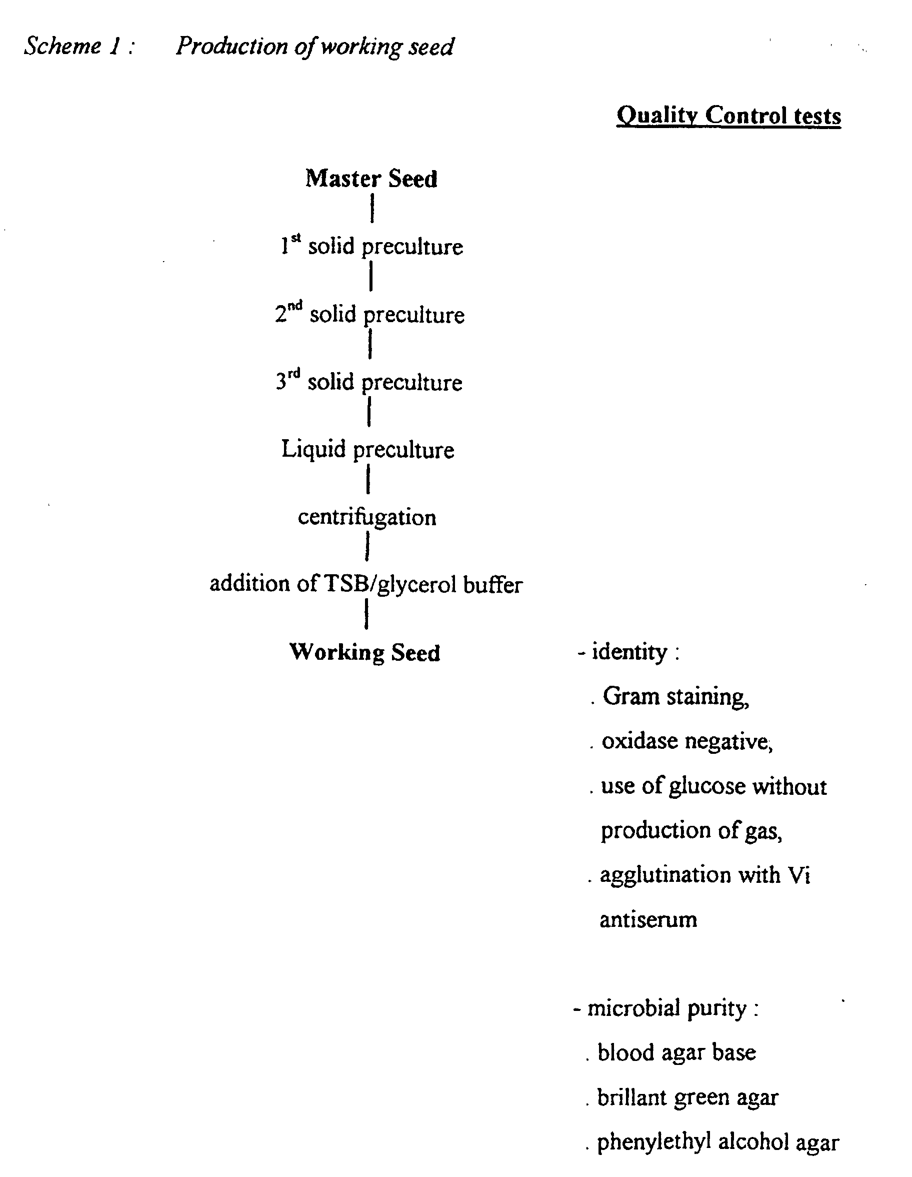

### 1. Growth on solid medium

The content of one vial of "Master Seed" (strain Saty 19430Ty2 obtained from ATCC) is thawed at room temperature and 0.2 ml of bacterial suspension is inoculated onto each of four Petri dishes containing 15 to 20 ml of solid Mueller-Hinton medium supplemented with 1% (v/v) of Polyvitex. This constitutes the first solid preculture. The remaining suspension of the "Master Seed" is used for an identity test.

After incubation at 36°C ± 2°C for 20 to 28 hours, one colony is picked on each Petri dish, which is then inoculated on each of four Petri dishes containing 15 to 20 ml of solid Mueller-Hinton medium. This constitutes the second solid preculture. An identity test is performed on the bacterial culture.

After incubation at 36°C ± 2°C for 20 to 28 hours, the bacterial growth in each Petri dish is resuspended in 3 ml of sterile saline solution. These are then transferred into each of four Roux bottles containing 100 ml of solid Mueller-Hinton medium. This constitutes the third solid preculture. Samples of the cell suspension are taken from each Petri dish for an identification test. The four Roux bottles are incubated at 36°C ± 2°C for 6 to 10 hours. The bacterial growth of each Roux bottle is resuspended in 6 ml of saline solution.

### 2. Liquid preculture

The 6 ml suspensions are transferred into each of four 3 litre flasks containing 0.9 L of liquid medium. They constitute the liquid preculture. The optical density (O.D.650 nm) of the liquid culture must be greater than 0.1 before incubation.

Samples are taken from each Roux bottle for identification testing. The flasks are placed on a shaking table (200 RPM) and incubated at 36°C ± 2°C for 12 to 20 hours after which the O.D.650nm must be superior to 0.2 (on 1/10 dilution). Samples are taken from each flask for testing of microbial purity.

### 3. Centrifugation

400 ml of liquid preculture is centrifuged under sterile conditions at 9000 RPM for 25 minutes. The supernatant is discarded and the pellets of each centrifugation bucket resuspended in 100 ml of TSB medium supplemented with 10% glycerol. The different suspensions are then pooled in a sterile recipient.

### 4. Distribution

The suspension is distributed under sterile conditions into polypropylene tubes (0.8 ml/tube) using an automatic syringe. Each tube is labelled and stored at -70°C. A total of 726 vials were prepared on 17/1/94 and constitute the working seed (19430 Ty2 17/01/94).

### 5. Control tests

The control tests performed on the different stages of the working seed are summarised in Scheme 1.

### Production of Vi polysaccharide

A summary of the manufacturing steps and Quality Control testing is shown in Scheme 2 below.

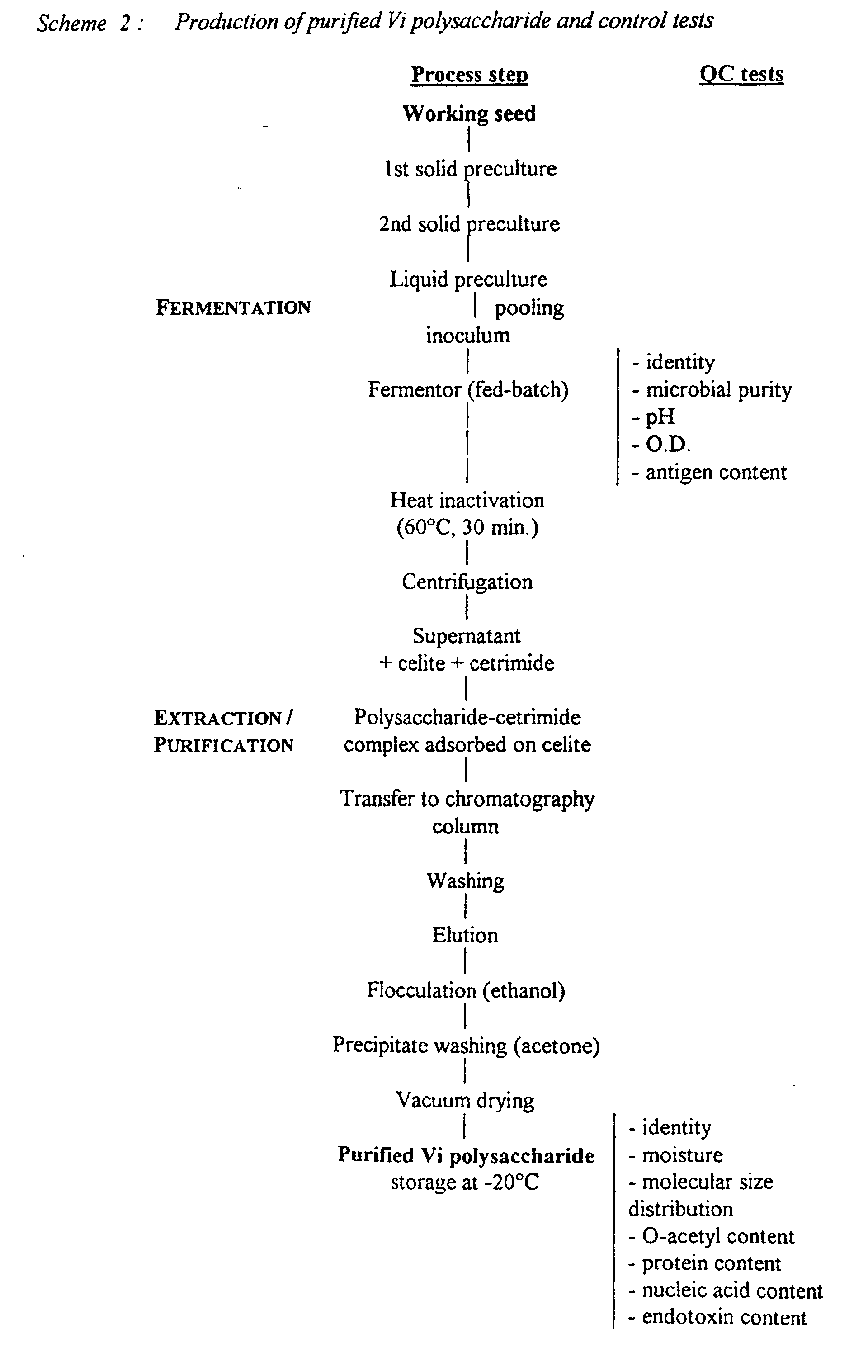

### A description of each step is given hereafter.

### 1. Fermentation

### 1.1. Growth on solid medium

The contents of a tube of working seed is thawed at room temperature and 0.3 ml of bacterial suspension is inoculated into each of three Petri dishes containing 15 to 20 ml of solid Mueller-Hinton medium. The working seed remaining in the tube is used for identity and microbial purity testing.

After incubation at 36°C ± 2°C for 20 to 28 hours, the surface growth of each Petri dish is resuspended in 4 ml of saline solution and 2 ml are transferred into one of six Roux bottles containing 100 ml of solid Mueller-Hinton medium. This constitutes the second solid preculture. Samples are taken from each Petri dish to be tested for microbial purity and identity. The six Roux bottles are incubated at 36°C ± 2°C for 6 to 10 hours.

### 1.2. Liquid preculture

The surface growth of each Roux bottle is resuspended in 10 ml of saline solution and transferred into each of six 3 L flasks containing 0.9 L of liquid medium. This constitute the liquid preculture.

The optical density (O.D.650 nm) is approximately 0.1 at start. Samples of the bacterial suspension are taken from each Roux bottle for purity and identity tests. The 6 flasks are placed on a shaking table (200 rpm) and incubated at 36°C ± 2°C for 12 to 20 hours. Liquid samples of each flask are taken for purity and identity tests before pooling the contents of 5 flasks (5 x 0.9 L). This volume constitutes the inoculum for the 200 L fermentor. The O.D. must be superior to 0.1 (on 1:10 dilution).

### 1.3. Batch fermentation

Prior to medium introduction, the fermenter is sterilised by steam. The medium is prepared in a separate tank and transferred to the fermentor trough a double filtration system for its sterilisation.

The inoculum is introduced into a 200 L (total volume) fermentor containing 120 to 140 L of liquid medium. The pH is adjusted to and maintained automatically at 7.2 by addition of sterile NaOH (10% w/v) or H3PO4 (10% w/v). The volumes added for pH correction do not exceed 2 litres for the acid and 10 litres for the caustic. The temperature is adjusted to and maintained at 36°C □ 1°C. The dissolved oxygen is maintained at 30%-50% saturation by control of aeration rate and agitation speed. An overpressure of 0.1 bar is maintained throughout fermentation in order to facilitate the oxygen transfer and to minimise foam formation. Sterile anti-foam (SAG 471) is added to the culture if too much foam is present. The volume of added anti-foam does not exceed 100ml.

Fed-batch cultivation is carried out by controlled addition of sterile feed medium (50% glucose). Aliquots of broth are taken at regular intervals throughout the exponential growth phase to follow the kinetics of microbial growth.

The total duration of fermentation is 8 to 14 hours and ends with the decrease of oxygen uptake rate. This corresponds to a minimum optical density (650 nm) of 0.1 as measured on a 1:100 dilution of the fermentation broth.

At the end of fermentation, a sample is taken for microbial identification/purity tests.

### 2. Extraction/purification

### 2.1. Heat inactivation

At the end of fermentation, the microbial suspension is immediately inactivated by heating the fermentor to 60°C □ 1°C for minimum 30 minutes under constant agitation. An aliquot (2 samples of about 30 ml) is taken in order to verify the efficacy of inactivation (no growth on appropriate culture medium with). The content of the fermentor is transferred into a sterile 200 L tank under sterile conditions and maintained at a temperature below 20°C until centrifugation.

### 2.2. Centrifugation

At the end of the inactivation process, the bacterial suspension is centrifuged in a semi-continuous sterilised centrifuge in order to eliminate the cellular debris. The supernatant is collected in an intermediate glass recipient in order to visualise its limpidity and is then transferred into a sterilised 200 L stainless steel tank. Collection rate during centrifugation is 35 to 55 litres/hour.

### 2.3. Complexation with cetrimide and fixation onto celite

A suspension of celite 545 (2.4 kg celite in 10 L of distilled water) and a 5% cetrimide solution are added successively to the supernatant of the centrifugation (130 L) in the 200 L stainless steel tank. The mixture is stirred with a propeller for at least 20 minutes in order to allow the formation of a polysaccharide-cetrimide complex which adsorbs onto the celite. The suspension is then left to decant for at least 20 minutes. The supernatant is eliminated by suction.
The complex adsorbed on celite is collected via a valve in the bottom of the tank and transferred into an apyrogenic chromatography column . The column is transferred into an explosion proof area.

### 2.4. Washing of the column

In order to eliminate adsorbed impurities, the celite is washed successively at room temperature, downflow, with the following solutions:
- 30 L of 0.05% cetrimide
- 30 L of 20% ethanol - 50 mM phosphate buffer, pH 6.0
- 40 L of 45% ethanol.
The flow is maintained between 0.75 - 1.25 L/min during the three steps.
All solutions are 0.22 µm filtered.

### 2.5. Elution

The polysaccharide is finally eluted at room temperature with a 50% ethanol/ 0.4 M NaCl 0.22 µm filtered solution. The eluate is collected in an apyrogenic glass flask. Elution is stopped when there is no more polysaccharide in the eluate (by precipitation test in 80 % ethanol + CaCl₂). The final volume of eluate is between 3 - 5 litres.

### 3. Flocculation

The eluate is transferred into a 10 or 20 L apyrogenic glass beaker. The polysaccharide is flocculated by addition of ethanol (volume added = volume eluate x 1.5). The suspension is stirred for minimum 20 minutes, then left to decant for at least 20 minutes. The supernatant is eliminated by suction. The suspension is centrifuged in the presence of an excess of ethanol. The operation is repeated a second time. The polysaccharide is collected on an apyrogenic fritted glass filter and washed with 1 L of acetone.

### 4. Drying

The polysaccharide is dried under vacuum at room temperature for at least 24 hours. After weighing, the polysaccharide is stored in an irradiated flask. Samples are removed for archiving and QC tests. The polysaccharide lot is labelled and stored at minus 20°C.

### Example 2: Concomitant Administration of Hepatitis A and Typhoid Fever Vaccines

For both typhoid fever and hepatitis A, major risk groups are travellers and workers moving from non-endemic to endemic countries and vaccination has been recognized as the only method providing long term protection against clinical disease. As both diseases share similar epidemiologies and risk groups, a logical step forward would be the simultaneous administration of vaccines against these diseases. This example describes two studies performed to evaluate the feasibility of simultaneous administrations of hepatitis A and Vi polysaccharide typhoid vaccines, by assessing the safety, reactogenicity and immunogenicity profiles.

### Materials and methods

### Study populations

Two independent studies were performed in two different study centres in healthy volunteers aged 18-50, with no medical history of hepatitis A and/or typhoid fever, and who had not received either *S. typhi* or hepatitis A vaccination in the previous 5 years. Local ethics committee approval from each study centre and written informed consent for each subject were obtained. Women of child-bearing age agreed to use appropriate contraception for the duration of the study.

Exclusion criteria included clinical signs of acute illness at time of study entry, any chronic treatment with immunosuppressive drugs including corticosteroids, any history of sensitivity to vaccine components, simultaneous participation in any other clinical trial, pregnancy, simultaneous administration of any other vaccine(s), administration of immunoglobulins within three weeks of enrolment or 2 months after vaccination. Also excluded were subjects found to be seropositive for hepatitis A, hepatitis B surface antigen, hepatitis C and/or anti-HIV antibodies at screening.

### Vaccines

All vaccines were prepared by SmithKline Beecham Biologicals (Rixensart, Belgium). Each 1 ml dose of the hepatitis A vaccine (Havrix-1440™), in vials or prefilled syringes, contained at least 1440 ELISA unit (EL.U) of the inactivated antigen adsorbed onto 0.5 mg aluminium (as AlOH₃). Each 0.5ml dose of typhoid vaccine, supplied in prefilled syringes, contained 25 µg Vi capsular polysaccharide. The combined vaccine contained 25µg Vi capsular polysaccharide and at least 1440 EL.U of the inactivated hepatitis A antigen adsorbed onto 0.5 mg aluminium (as AlOH₃) in 1 ml monodose vials.

### Study design

Both studies were open, randomised studies in which vaccines were administered on day 0 as either one injection (monovalent, mixed and combined) or two injections (concomitant) in separate arms. Subjects recorded solicited and unsolicited signs and symptoms on diary cards until day 4 with subject follow-up until day 28. Blood samples were drawn on days 0 and 28 for determination of anti-HAV and anti-Vi antibody titres.

In study 1, performed at the Clinique Notre Dame de Grâce, Gosselies, Belgium, two groups of 50 subjects each, received either concomitant vaccination of both vaccines in separate arms, or a single injection of the two vaccines mixed extemporaneously (1.5ml volume) in one syringe. There was no significant difference between groups with respect to the distribution of males and females (p = 0.69) or with respect to mean ages between males and females (p = 0.48), between groups (p = 0.17) or for the group/sex interaction (p = 0.08).

In the second study performed at the University Hospital of Hradec Kralové (Czech Republic), three groups of 100 subjects each, received either one injection of hepatitis A or typhoid vaccines alone, or the combined vaccine and one group of 101 subjects received both vaccines concomitantly in separate arms. There was no significant difference between groups with respect to the male/female ratio (p = 0.798). There was a difference in mean ages (p = 0.003) between males and females which was not considered to be clinically relevant, but not between groups (p = 0.803) nor for the group/sex interaction (p = 0.770). For the purposes of the study, the groups were considered comparable.

### Assessment of safety and reactogenicity

Solicited local adverse events (erythema and swelling) were described by the measurement of the longest diameter. Injection site soreness and solicited general adverse events, fever, malaise, nausea, headache, general aches, and itching were graded by the subjects. Any adverse event which prevented normal everyday activities and necessitated a corrective therapy was defined as severe.

### Serology

Pre- and post-vaccination sera were analysed in a blinded fashion at SmithKline Beecham Biologicals (Rixensart, Belgium). Anti-HAV antibodies were determined using a commercial ELISA kit (Enzymun, Boehringer) with a cut-off value of 33 mIU/ml. Subjects were considered to have seroconverted if they showed an increase in anti-HAV titre from < 33 mIU/ml (seronegative) to ≥ 33 mIU/ml (seropositive).
Anti-Vi polysaccharide titres were determined using an in-house ELISA, with an assay cut-off at 150 EL.U/ml, corresponding to approximately 3 times the lower quantitation limit of the assay. Subjects with pre-vaccination titers < 150 EL.U./ml seroconverted when their post-vaccination titre was ≥150 EL.U./ml.

### Statistical Methods:

A two-way ANOVA (analysis of variance) was used to compare mean ages between groups and sexes; Fisher's exact test to compare distribution of males to females. Geometric mean antibody titres (GMTs) and seroconversion rates (SCs) of anti-HAV and anti-Vi polysaccharide antibodies were calculated. GMT titres below the assay cut-off (anti-HAV antibody titre <33 mIU/ml and anti-Vi antibody titre <150 EL.U/ml) were given an arbitrary value of half the cut-off. A one-way ANOVA was used to compare GMTs between groups.

### Results

### Reactogenicity

The majority of adverse events reported in both studies (Tables 1 and 2) were local, mild to moderate in intensity and transient. No serious adverse events were reported in either study and all adverse events resolved without sequelae.

The incidence of subjects reporting symptoms are shown in Table 1. In study 1, there was no clinically relevant difference in the number of subjects who reported local and general symptoms when both the hepatitis A and Vi polysaccharide vaccines were injected concomitantly (64%) or after mixing (56%). In study 2, similar incidences of symptoms were reported following concomitant vaccination with both vaccines or the combined vaccine (66% vs. 67%, respectively), while fewer reports were associated with the separately injected hepatitis A or typhoid monovalent vaccines (56% and 36%, respectively). General symptoms were infrequent, mainly mild in intensity and reported with similar frequency in all groups in each study (24% in study 1 and 24-30% in study 2). Subjects who received either the hepatitis A vaccine alone or co-administered with the typhoid vaccine, reported more local symptoms than those who received the monovalent typhoid vaccine (44-59% vs. 10-22%).

Mild to moderate injection site soreness was the most frequently reported local symptom (Table 2). In study 1, one subject per group (mixed and concomitant/separate- Vi arm) reported erythema > 30mm and severe soreness. In study 2 only one case of swelling > 30mm with monovalent hepatitis A vaccine was reported. Headache, all mild to moderate in intensity, was the most frequently reported general symptom. The only general symptom graded as severe by the investigator because it prevented normal day activity was one case of general aches, suspected to be related to vaccination following concomitant administration of both vaccines in study 2. However this did not require any corrective therapy.

### Immunogenicity

Almost all subjects seroconverted one month after vaccination with respect to both HAV and Vi antibodies (94.4-100%) (Table 3).

In study 1, similar immune responses were induced against both antigens, with no effect due to the mode of administration (mixed vs. concomitant administration, GMT = 1159 EL.U/ml and 1331 EL.U/ml, respectively for anti-Vi and GMT = 302 EL.U/ml and 367 EL.U/ml, respectively for anti-HAV). Seroconversion rates were > 95.6% in all cases.

In study 2, GMTs following vaccination with either vaccine alone, both vaccines administered concomitantly or as a combined vaccine (anti-Vi: 1307, 1247 and 942 EL.U/ml, respectively; anti-HAV: 462, 517 and 432, respectively) were not significantly different (p = 0.45 for anti-HAV, p = 0.18 for anti-Vi). Seroconversion rates were > 94.4% in all cases.

**Table 1:**

| **Percentages of subjects reporting symptoms (local and/or general)** | | | | | | |
|---|---|---|---|---|---|---|
| | **Study 1** | | **Study 2** | | | |
| **Vaccine** | Mixed | Separate arms | HAV alone | Vi alone | Separate arms | Combined |
| | N=50 | N=50 | N=100 | N=100 | N=100 | N=100 |
| | **(%)** | **(%)** | **(%)** | **(%)** | **(%)** | **(%)** |
| Overall | 56 | 64 | 56 | 36 | 66 | 67 |
| General | 24 | 24 | 28 | 24 | 27 | 30 |
| Local | 44 | 50 (HAV) | 50 | - | 49 (HAV) | 59 |
| | - | 22 (Vi) | - | - | 19 (Vi) | |
| Overall = Percentage of subjects reporting at least one symptom. Some subjects may have reported more than one symptom. | | | | | | |
| General = Percentage of subjects reporting at least one general symptom | | | | | | |
| Local = Percentage of subjects reporting at least one local symptom | | | | | | |
| HAV = at hepatitis A vaccine site | | | | | | |
| Vi = at typhoid vaccine site | | | | | | |
| N = Number of subjects | | | | | | |

**Table 2:**

| **Incidence of solicited general and local symptoms as percentage of subjects** | | | | | | | |
|---|---|---|---|---|---|---|---|
| | | **Study 1** | | **Study 2** | | | |
| | **Vaccine** | Mixed | Separate arms | HAV alone | Vi alone | Separate arms | Combined |
| | | N=50 | N=50 | N=100 | N=100 | N=100 | N=100 |
| | | **(%)** | **(%)** | **(%)** | **(%)** | **(%)** | **(%)** |
| **General Symptoms** | General aches | 6 | 0 | 2 | 1 | 5 | 9 |
| | Headache | 7 | 10 | 16 | 9 | 12.9 | 14 |
| | Itching | 0 | 2 | 2 | 1 | 4.0 | 1 |
| | Malaise | 4 | 2 | 17 | 13 | 11.9 | 15 |
| | Nausea | 2 | 0 | 2 | 7 | 5.9 | 5 |
| | Fever | 0 | 4 | 1 | 0 | 2.0 | 0 |
| **Local Symptoms** | Erythema | 80 | 2 (HAV) | 12 | 11 | 10.9 (HAV) | 6 |
| | | | 6 (Vi) | | | 6.9 (Vi) | |
| | Soreness | 40 | 48 (HAV) | 45 | 5 | 46.5 (HAV) | 58 |
| | | | 16 (Vi) | | | 13.9 (Vi) | |
| | Swelling | 4 | 4 (HAV) | 2 | 1 | 4.0 (HAV) | 3 |
| | | | 4 (Vi) | | | 1.0 (Vi) | |
| HAV = at hepatitis A vaccine site | | | | | | | |
| Vi = at typhoid vaccine site | | | | | | | |
| N = Number of subjects | | | | | | | |
| NB. Some subjects may have reported more than one symptom. | | | | | | | |

**Table 3:**

| **Immune responses of subjects, one month post-vaccination** | | | | | |
|---|---|---|---|---|---|
| | | **Anti-Vi** | | **Anti-HAV** | |
| | **Group** | **SC (%)** | **GMT** | **SC (%)** | **GMT** |
| **Study 1** | HAV and Vi | 95.6 | 1159 | 97.9 | 302 |
| | Mixed | (N=45) | (813-1652) | (N=47) | (217-421) |
| | HAV and Vi | 100 | 1331 | 98.0 | 367 |
| | Separate arms | (N=44) | (943-1878) | (N=49) | (268-502) |
| | HAV alone | - | - | 100.0 | 462 |
| | | | | (N=97) | (385-553) |
| **Study 2** | Vi alone | 94.4 | 1307 | - | - |
| | | (N=90) | (1001-1707) | | |
| | HAV and Vi | 95.5 | 1247 | 97.9 | 517 |
| | Separate arms | (N=89) | (961-1617) | (N=96) | (415-645) |
| | HAV and Vi | 96.0 | 942 | 98.9 | 432 |
| | Combined | (N=75) | (734-1209) | (N=95) | (351-531) |
| Anti-Vi = antibody against Vi polysaccharide typhoid antigen | | | | | |
| Anti-HAV = antibody against hepatitis A antigen | | | | | |
| SC (%) = Seroconversion rate; % of subjects with anti-Vi titers ≥ 150 EL.U/ml or anti-HAV titers ≥ 33 EL.U/ml | | | | | |
| N = Number of subjects | | | | | |
| GMT = Geometric Mean Titre (EL.U/ml) with 95% confidence interval in parentheses | | | | | |

### Discussion

These results show that Havrix-1440™ can be successfully co-administered with SmithKline Beecham Biologicals' candidate Vi polysaccharide typhoid vaccine to healthy adults as a newly formulated combined vaccine. The vaccines were highly immunogenic, with seroconversion rates > 94% against both components, and there was no cross-interference in the immune profiles, subjects seroconverting to both antigens to the same extent as the monovalent vaccines.

The mode of administration did not affect the safety, reactogenicity or immunogenicity of the respective vaccines. The coadministration of both vaccines did not significantly affect the frequency and intensity of symptoms. Similar incidences of symptoms were reported by subjects vaccinated with the hepatitis A vaccine, either alone or coadministered with the typhoid vaccine, and there were fewer reports for the typhoid vaccine alone. Mild to moderate injection site soreness was the most frequently reported symptom, in agreement with published literature for Havrix™. The larger volume (1.5 ml), when the two vaccines were mixed in one syringe, did not result in an increased reporting of local symptoms when compared to the hepatitis A vaccine. Indeed, fewer local symptoms were reported following the administration of the extemporaneously mixed vaccines than for hepatitis A vaccine alone (44% vs. 50%). General symptoms were infrequent, mainly mild in intensity and reported with similar frequency in all groups.

### Example 3: An immunogenicity experiment with a combined Vi polysaccharide typhoid and an inactivated hepatitis A vaccine.

### Methods

A multi-centre study evaluated the longer term follow-up of a consistency study of 3 lots of combined Vi typhoid and hepatitis A vaccine. For the consistency study 462 healthy subjects, aged 15-50 years, were vaccinated. The single dose of vaccine contains 25µg typhoid Vi polysaccharide and >1440 ELISA units of inactivated hepatitis A (1ml dose). During the consistency study the safety and bioequivalence of the 3 vaccine lots was demonstrated. At month 6 the vaccinees were offered a booster dose of SB Bio's hepatitis A vaccine and a randomised subset was followed for immunogenicity.

### Results:

**Table 4**

| | Anti-HAV | | | Anti-Vi | | |
|---|---|---|---|---|---|---|
| Time | N | % SP | GMT | N | % SP | GMT |
| Day 14 | 127 | 89.8 | 157.5 | 118 | 97.5 | 1260.2 |
| Month 1 | 397 | 99.0 | 452.4 | 374 | 95.7 | 1022.2 |
| Month 6 | 141 | 95.0 | 150.3 | 128 | 82.0 | 569.0 |
| Month 7 | 141 | 100 | 3392.0 | 131 | 80.9 | 528.9 |
| GMT (geometric mean titre) is in mIU/ml (HAV) and EL.U/ml (Vi), | | | | | | |
| SP = seroconversion (titres ≥33 mIU/ml (HAV) and ≥150 EL.U/ml (Vi)). | | | | | | |

### Conclusion

The combined vaccine against typhoid fever and hepatitis A elicits a good immune response with rapid initial seroconversion and persitence of SP% between 82.0% (Vi) and 95.0% (HAV) up to month 6. One month after a booster dose of hepatitis A vaccine all vaccinees are immune for hepatitis A and 7 months after the initial vaccination still >80% remain immune for typhoid fever. The combined vaccine is safe and well tolerated in healthy adults and adolescents (15-18 years of age).

### Example 4: Further Confirmation of the feasibility of a combined hepatitis A and typhoid fever vaccine.

A Phase II open randomised study was performed in 401 healthy adults aged 18-50 years. About 100 subjects per group received a single dose of candidate combined Vi polysaccharide and hepatitis A vaccine, or the Vi polysaccharide typhoid vaccine (Typherix™) alone, or the hepatitis A vaccine (Havrix-1440™) alone or both monovalent vaccines concomitantly at month 0. The reactogenicity and immunogenicity profiles of the combined vaccine were evaluated and compared to that of the monovalent vaccines administered alone or concomitantly.

At month 12, a second, booster dose of the combined vaccine was given to subjects previously vaccinated with Havrix alone (group 1). A second dose of Havrix was also given to subjects who had received the combined vaccine or Havrix and Vi concomitantly.

### Safety and Reactogenicity

The incidence of symptoms reported during the 5 day follow-up period after vaccination was as follows. Subjects who received the hepatitis A vaccine either alone or in combination with the Vi polysaccharide typhoid vaccine reported more symptoms than the recipients of the Vi vaccine. Most of the reported symptoms were local in nature. A similar incidence of symptoms was observed when the hepatitis A and Vi vaccines were administered concomitantly in different arms or as a combined vaccine.

There were fewer reports of symptoms after the booster as compared with primary vaccination, regardless of which vaccine combination they received.

The incidence of local and general symptoms after primary and booster vaccination was as follows.

Soreness at the site of injection was the most frequently reported local symptom (after primary and booster vaccination) and the incidence was highest in recipients of Havrix with or without the Vi vaccine. One case of swelling was reported as grade '3' (> 30 mm and lasting over 24 hours). All other cases were mild to moderate in intensity. General symptoms were infrequent and mild in intensity and reported with lower frequency after booster vaccination compared with primary vaccination. The most commonly reported symptom after the primary vaccination was headache, and malaise and headache after the booster. Only one report (after dose 1), of general aches suspected of being related to vaccination was graded as '3'. Approximately 75% of all general symptoms reported were considered as being probably associated with or suspected of being related to vaccination.

The incidence of adverse events was not correlated with the sequence of vaccination (i.e. HA followed by HA-Vi or vice versa). All solicited symptoms resolved spontaneously.

### Immunogenicity

The immune responses following vaccination are shown in Table 5. All subjects were initially seronegative for anti-Vi and anti-HAV antibody titres.

### Anti-Vi -response after one dose of vaccine

Similar seroconversion rates to anti Vi were observed for subjects in group 2, 3 & 4. A significant difference in GMTs could not be shown between groups receiving the Vi polysaccharide vaccine either concomitantly or combined with the inactivated hepatitis A vaccine or alone (p = 0.13 for group 2 vs group 3 and p = 0.08 for group 3 vs group 4 by Student's t test).

### Persistence of anti-Vi - antibodies

Anti-Vi - persistence of antibodies was measured in groups 2, 3 & 4. Twelve months after one dose of vaccine, slightly lower immune results were obtained in group 3, but confidence intervals were large and overlapping. Seroconversion rates had decreased by 1.2-1.6 fold and GMTs 3 to 4 fold from the month 1 levels. Overall, 60%-76% of all subjects remained seropositive with GMTS between 240-394 EL.U/ml.

### Anti-HAV response to vaccination

Subjects in group 1 received the hepatitis A vaccine followed by the combined HA-Vi vaccine, group 2 received the Vi vaccine concomitantly with the hepatitis A vaccine followed by the hepatitis A vaccine, and group 3 received the combined HA-Vi vaccine followed by the hepatitis A vaccine. Similar seropositivity rates to anti HAV were observed after dose 1 (98%-100%). A significant difference in GMTs could not be shown between groups receiving the hepatitis A vaccine either simultaneously or combined with the Vi polysaccharide vaccine or alone (p = 0.61 for group 1 vs group 3 and p = 0.19 for group 2 vs group 3 by Student's t test).

### Anti-HAV - persistence of antibodies and effect of a booster

Immediately prior to the booster dose at month 12, anti-HAV antibodies had persisted in 88.3%, 92.5% and 91.5% of subjects, and GMTs were 79.6, 85.2 and 81.8 mIU/ml in groups 1, 2 & 3 respectively. GMTs had decreased by approximately 80% from the month 1 levels. All subjects tested one month after the booster dose were seropositive with similar levels of GMTs. GMTs had increased between 29 and 33-fold as compared to pre booster values.

**Table 5:**

| **Seroconversion/seropositivity rates (%) and geometric mean titres. (GMT) of anti-HAV antibody (according to protocol analysis)** | | | | | | | | |
|---|---|---|---|---|---|---|---|---|
| Timing | N | S+ | % | 95% CI Lower-Upper | | GMT | 95% CI Lower-Upper | |
| Group 1:Havrix and HA-Vi | | | | | | | | |
| Pre | 97 | 0 | 0.0 | 0.0 | 3.7 | 16.5 | 16.5 | 16.5 |
| PI(ml) | 97 | 97 | 100.0 | 96.3 | 100.0 | 461.5 | 385.1 | 553.0 |
| PI(m3) | 97 | 91 | 93.8 | 87.0 | 97.7 | 126.2 | 105.8 | 150.5 |
| PI(m6) | 95 | 79 | 83.2 | 74.1 | 90.1 | 83.1 | 67.4 | 102.3 |
| PI(m9) | 95 | 81 | 85.3 | 76.5 | 91.7 | 82.4 | 67.4 | 100.8 |
| PI(m12) | 94 | 83 | 88.3 | 80.0 | 94.0 | 79.6 | 66.0 | 96.0 |
| PII(m13) | 94 | 94 | 100.0 | 96.2 | 100.0 | 2692.2 | 2230.0 | 3250.3 |

| Group 2: Ha + Vi and | | | | | | | | |
|---|---|---|---|---|---|---|---|---|
| Havrix | | | | | | | | |
| Pre | 96 | 0 | 0.0 | 0.0 | 3.8 | 16.5 | 16.5 | 16.5 |
| PI(ml) | 96 | 94 | 97.9 | 92.7 | 99.7 | 517.3 | 414.9 | 645.1 |
| PI(m3) | 96 | 92 | 95.8 | 89.7 | 98.9 | 147.9 | 124.4 | 175.7 |
| PI(m6) | 96 | 87 | 90.6 | 82.9 | 95.6 | 98.4 | 81.2 | 119.2 |
| PI(m9) | 95 | 82 | 86.3 | 77.7 | 92.5 | 83.9 | 68.7 | 102.6 |
| PI(m12) | 93 | 86 | 92.5 | 85.1 | 96.9 | 85.2 | 70.8 | 102.5 |
| PII(m13) | 93 | 93 | 100.0 | 96.1 | 100.0 | 2487.9 | 2064.3 | 2998.6 |

| Group 3: HA-Vi and Havrix | | | | | | | | |
|---|---|---|---|---|---|---|---|---|
| Pre | 95 | 0 | 0.0 | 0.0 | 3.8 | 16.5 | 16.5 | 16.5 |
| PI(ml) | 95 | 94 | 98.9 | 94.3 | 100.0 | 431.5 | 350.6 | 531.1 |
| PI(m3) | 95 | 93 | 97.9 | 92.6 | 99.7 | 142.7 | 121.1 | 168.1 |
| PI(m6) | 95 | 80 | 84.2 | 75.3 | 90.9 | 90.7 | 73.8 | 111.4 |
| PI(m9) | 94 | 79 | 84.0 | 75.0 | 90.8 | 81.3 | 67.2 | 98.4 |
| PI(m12) | 94 | 86 | 91.5 | 83.9 | 96.3 | 81.8 | 69.1 | 96.8 |
| PII(m13) | 94 | 94 | 100.0 | 96.2 | 100.0 | 2581.8 | 2210.5 | 3015.6 |
| Comparison of anti-HAV GMTs at ml: G1 vs G3: p = 0.61 and G2 vs G3: p = 0.19 by Student's t test. | | | | | | | | |
| Notes: N = total number of documented doses | | | | | | | | |
| n = documented doses with at least one report of a symptom after vaccination | | | | | | | | |
| PB/SU = probably related or suspected to be related to vaccination | | | | | | | | |
| Group 1: HA followed by HA-Vi, Group 2: HA + Vi followed by HA, | | | | | | | | |
| Group 3: HA-Vi followed by HA. Anti-HAV not tested in group 4 (recipients of Vi only). | | | | | | | | |

The results of this study confirm that the candidate combined hepatitis A and Vi polysaccharide typhoid vaccine is safe and well tolerated in healthy adults. There was no significant difference in GMTs between the combined Vi polysaccharide typhoid and hepatitis A vaccine and Typherix™ or Havrix™. Similar seropositivity rates after vaccination, and slightly lower persistence of antibodies up to 12 months after vaccination were also observed.

A booster effect (seropositivity and GMTs) on anti-HAV antibodies was observed when either the combined vaccine was used to boost HavrixTM or vice versa, and when Havrix™ was used to boost titres following concomitant administration of Havrix™ and Typherix™.

### Conclusions

These findings show that the candidate combined hepatitis A and Vi polysaccharide typhoid vaccine is safe, well tolerated and immunogenic in all populations evaluated. It is comparable in terms of its reactognicity profile, immunogenicity and antibody persistence to the existing commercially available monovalent vaccines (Typherix™ and Havrix ™ 1440). The vaccine can be safely integrated into a vaccination schedule for hepatitis A.

## Claims

1. A vaccine composition comprising:
(a) a *Salmonella typhi* purified Vi polysaccharide,
(b) a hepatitis A antigen; and
(c) an aluminium salt
wherein the purification of the Vi polysaccharide does not contain phenol, such that:
when the Vi polysaccharide is combined with a hepatitis A antigen in solution the Vi retains stability and the hepatitis A antigen is not affected by the detrimental effects of phenol; and
the vaccine components are stable and do not interfere with each other.

2. A vaccine composition according to claim 1 wherein vaccinees seroconvert to both *Salmonella typhi* Vi polysaccharide and hepatitis A antigen to same extent as the monovalent components.

3. A vaccine according to claim 1 or 2 wherein the Vi polysaccharide is stabilised by dehydrating with buffer in the absence of phenol.

4. A vaccine composition according to any preceding claim obtained by extemporaneous mixing of a hepatitis A vaccine and a typhoid vaccine in one syringe.

5. A vaccine composition according to any preceding claim which additionally comprises an adjuvant.

6. A vaccine composition according to claim 5 wherein the adjuvant is a preferential stimulator of TH1-cell response.

7. A vaccine composition according to any preceding claim which additionally comprises a carrier.

8. A vaccine composition according to claim 6 in which the preferential stimulator of TH1-cell response is selected from the group of adjuvants comprising: 3D-MPL, 3D-MPL wherein the size of the particles of 3D-MPL is preferably about or less than 100nm, QS21, a mixture of QS21 and cholesterol, or a combination of two or more of said adjuvants.

9. A vaccine composition according to claim 8 in which the preferential stimulator of TH1-cell response is 3D-MPL.

10. A vaccine composition according to any one of claims 1 to 9 in which the Hepatitis A antigen is derived from the HM-175 strain.

11. A vaccine composition according to any preceding claim in which an hepatitis B antigen is additionally present.

12. A vaccine composition as defined in claim 11 in which the Hepatitis B antigen is hepatitis surface antigen.

13. A vaccine composition according to any preceding claim wherein the aluminium salt is aluminium hydroxide.

14. A vaccine composition according to claim 7 in which the carrier is selected from the group comprising aluminium phosphate and an oil in water emulsion.

15. A vaccine composition according to any preceding claim which additionally comprises a dengue antigen.

16. A vaccine composition according to claim 15 in which the dengue antigen is selected from the group comprising envelope (E) glycoprotein proteins, truncated envelope glycoprotein proteins and Dengue viral proteins.

17. A vaccine composition according to any preceding claim which additionally comprises an hepatitis E antigen.

18. A vaccine composition according to claim 17 in which the hepatitis E antigen is SAR 55.

19. Use of a Vi polysaccharide in the preparation of a combination vaccine, the vaccine comprising a salmonella typhi purified Vi polysaccharide, a hepatitis A antigen and an aluminium salt, wherein the Vi polysaccharide is prepared in the absence of phenol.

20. A process for the production of a combination vaccine, the vaccine comprising Salmonella typhi purified Vi polysaccharide, a hepatitis A antigen and an aluminium salt, the process comprising preparation of a *Salmonella typhi* Vi polysaccharide in the absence of phenol.

21. A process according to claim 20, wherein the polysaccharide is stabilised by dehydrating with buffer in the absence of phenol.

22. A process according to claim 20 or 21, wherein the *Salmonella typhi Vi* polysaccharide is administered with an inactivated hepatitis A antigen.

23. Use of a *Salmonella typhi* Vi polysaccharide prepared the absence of phenol in the preparation of a combination vaccine, the vaccine comprising a Salmonella typhi purified Vi polysaccharide, a hepatitis A antigen and an aluminium salt.

24. Use according to claim 23, wherein the combination vaccine is against typhoid fever and hepatitis A.

## Revendications

1. Composition vaccinale comprenant :
- (a) un polysaccharide Vi purifié de *Salmonella typhi*,
- (b) un antigène de l'hépatite A ; et
- (c) un sel d'aluminium,
dans laquelle la purification du polysaccharide Vi ne contient pas de phénol, de sorte que : lorsque le polysaccharide Vi est combiné avec un antigène de l'hépatite A en solution, le Vi reste stable et l'antigène de l'hépatite A n'est pas affecté par les effets préjudiciables du phénol ; et les composants du vaccin sont stables et n'interfèrent pas les uns avec les autres.

2. Composition vaccinale selon la revendication 1, dans laquelle les vaccinés séroconvertissent à la fois le polysaccharide Vi purifié de *Salmonella typhi*, et l'antigène de l'hépatite A au même niveau qu'avec les composants monovalents.

3. Vaccin selon la revendication 1 ou la revendication 2, dans lequel le polysaccharide Vi est stabilisé par déshydratation du tampon en l'absence de phénol.

4. Composition vaccinale selon l'une quelconque des revendications précédentes, obtenue par le mélange extemporané d'un vaccin contre l'hépatite A et d'un vaccin contre la typhoïde, dans une seule seringue.

5. Composition vaccinale selon l'une quelconque des revendications précédentes qui comprend en plus un adjuvant.

6. Composition vaccinale selon la revendication 5, dans laquelle l'adjuvant est un stimulateur préférentiel de la réponse des cellules TH1.

7. Composition vaccinale selon l'une quelconque des revendications précédentes, qui comprend en plus, un véhicule.

8. Composition vaccinale selon la revendication 6, dans laquelle le stimulateur préférentiel de la réponse des cellules TH1 est choisi au sein du groupe des adjuvants comprenant le 3D-MPL, le 3D-MPL dans lequel la taille des particules de 3D-MPL est de préférence inférieure à 100 nm, le QS21, un mélange de QS21 et de cholestérol ou une combinaison de deux ou plusieurs adjuvants.

9. Composition vaccinale selon la revendication 8, dans laquelle le stimulateur préférentiel de la réponse des cellules TH1 est le 3D-MPL.

10. Composition vaccinale selon l'une quelconque des revendications 1 à 9, dans laquelle l'antigène de l'hépatite A est dérivé de la souche HM-175.

11. Composition vaccinale selon l'une quelconque des revendications précédentes, dans laquelle un antigène de l'hépatite B est présent en plus.

12. Composition vaccinale selon la revendication 11, dans laquelle l'antigène de l'hépatite B est l'antigène de surface de l'hépatite B.

13. Composition vaccinale selon l'une quelconque des revendications précédentes, dans laquelle le sel d'aluminium est l'hydroxyde d'aluminium.

14. Composition vaccinale selon la revendication 7, dans laquelle le véhicule est choisi au sein du groupe comprenant le phosphate d'aluminium et une émulsion huile-dans-l'eau.

15. Composition vaccinale selon l'une quelconque des revendications précédentes, qui comprend en plus, un antigène de la dengue.

16. Composition vaccinale selon la revendication 15, dans laquelle l'antigène de la dengue est choisi au sein du groupe comprenant les protéines glycoprotéiques d'enveloppe (E), les protéines glycoprotéiques d'enveloppe tronquées et les protéines virales de la dengue.

17. Composition vaccinale selon l'une quelconque des revendications précédentes, qui comprend en plus, un antigène de l'hépatite E.

18. Composition vaccinale selon la revendication 17, dans laquelle l'antigène de l'hépatite E est le SAR 55.

19. Utilisation d'un polysaccharide Vi dans la préparation d'un vaccin combiné, le vaccin comprenant un polysaccharide Vi purifié de *Salmonella typhi*, un antigène de l'hépatite A et un sel d'aluminium, dans laquelle le polysaccharide Vi est préparé en l'absence de phénol.

20. Procédé pour la production d'un vaccin combiné, le vaccin comprenant un polysaccharide Vi purifié de *Salmonella typhi*, un antigène hépatite A et un sel d'aluminium, le procédé comprenant la préparation d'un polysaccharide Vi de *Salmonella typhi* en l'absence de phénol.

21. Procédé selon la revendication 20, dans lequel le polysaccharide est stabilisé par déshydratation avec du tampon en l'absence de phénol.

22. Procédé selon la revendication 20 ou la revendication 21, dans lequel le polysaccharide de *Salmonella typhi* Vi est administré avec un antigène de l'hépatite A inactivé.

23. Utilisation d'un polysaccharide de *Salmonella typhi* Vi préparé en l'absence de phénol, dans la préparation d'un vaccin combiné, le vaccin comprenant un polysaccharide Vi purifié de *Salmonella typhi*, un antigène de l'hépatite A et un sel d'aluminium.

24. Utilisation selon la revendication 23, dans laquelle le vaccin combiné est dirigé contre la fièvre typhoïde et l'hépatite A.

## Patentansprüche

1. Impf Stoffzusammensetzung, umfassend:
(a) ein gereinigtes Salmonella typhi-Vi-Polysaccharid,
(b) ein Hepatitis A-Antigen; und
(c) ein Aluminiumsalz,
worin die Reinigung des Vi-Polysaccharids kein Phenol enthält, so daß: wenn das Vi-Polysaccharid mit einem Hepatitis A-Antigen in Lösung kombiniert wird, das Vi Stabilität beibehält und das Hepatitis A-Antigen nicht durch die nachteiligen Wirkungen von Phenol beeinträchtigt wird; und
die Impfstoff-Komponenten stabil sind und sich nicht gegenseitig beeinträchtigen.

2. Impfstoffzusammensetzung gemäß Anspruch 1, worin die Impflinge zu sowohl Salmonella typhi-Vi-Polysaccharidals auch Hepatitis A-Antigen im gleichen Ausmaß wie die monovalenten Komponenten serokonvertieren.

3. Impfstoff gemäß Anspruch 1 oder 2, worin das Vi-Polysaccharid durch Dehydratisieren mit Puffer in Abwesenheit von Phenol stabilisiert ist.

4. Impfstoffzusammensetzung gemäß einem der vorhergehenden Ansprüche, erhalten durch unvorbereitetes Vermischen eines Hepatitis A-Impfstoffs und eines Typhus-Impfstoffs in einer Spritze.

5. Impfstoffzusammensetzung gemäß jedem vorhergehenden Anspruch, die zusätzlich ein Adjuvans umfaßt.

6. Impfstoffzusammensetzung gemäß Anspruch 5, worin das Adjuvans ein bevorzugter Stimulator der TH1-Zellreaktion ist.

7. Impfstoffzusammensetzung gemäß jedem vorhergehenden Anspruch, die zusätzlich einen Träger umfaßt.

8. Impfstoffzusammensetzung gemäß Anspruch 6, worin der bevorzugte Stimulator der TH1-Zellreakton aus der Gruppe von Adjuvantien ausgewählt ist, die umfaßt: 3D-MPL, 3D-MPL, worin die Größe der Teilchen von 3D-MPL bevorzugt ca. oder weniger als 100 nm ist, QS21, eine Mischung aus QS21 und Cholesterol, oder eine Kombination aus zwei oder mehreren der Adjuvantien.

9. Impfstoffzusammensetzung gemäß Anspruch 8, worin der bevorzugte Stimulator der TH1-Zellreaktion 3D-MPL ist.

10. Impfstoffzusammensetzung gemäß einem der Ansprüche 1 bis 9, worin das Hepatitis A-Antigen aus dem HM-175-Stamm abstammt.

11. Impfstoffzusammensetzung gemäß jedem vorhergehenden Anspruch, worin zusätzlich ein Hepatitis B-Antigen vorhanden ist.

12. Impfstoffzusammensetzung wie in Anspruch 11 definiert, in der das Hepatitis B-Antigen ein Hepatitis-Oberflächenantigen ist.

13. Impfstoffzusammensetzung gemäß jedem vorhergehenden Anspruch, worin das Aluminiumsalz Aluminiumhydroxid ist.

14. Impfstoffzusammensetzung gemäß Anspruch 7, in der der Träger aus der Gruppe ausgewählt ist, die Aluminiumphosphat und eine Öl-in-Wasser-Emulsion umfaßt.

15. Impfstoffzusammensetzung gemäß jedem vorhergehenden Anspruch, die zusätzlich ein Dengue-Antigen umfaßt.

16. Impfstoffzusammensetzung gemäß Anspruch 15, in der das Dengue-Antigen aus der Gruppe ausgewählt ist, die Hüll-(E)-Glycoprotein-Proteine, abgestumpfte HüllGlycoprotein-Proteine und virale Dengue-Proteine umfaßt.

17. Impfstoffzusammensetzung gemäß jedem vorhergehenden Anspruch, die zusätzlich ein Hepatitis E-Antigen umfaßt.

18. Impfstoffzusammensetzung gemäß Anspruch 17, in der das Hepatitis E-Antigen SAR55 ist.

19. Verwendung eines Vi-Polysaccharids in der Herstellung eines Kombinationsimpfstoffs, wobei der Impfstoff ein gereinigtes Salmonella typhi-Vi-Polysaccharid, ein Hepatitis A-Antigen und ein Aluminiumsalz umfaßt, worin das Vi-Polysaccharid in Abwesenheit von Phenol hergestellt wird.

20. Verfahren zur Herstellung eines Kombinationsimpfstoffs, wobei der Impfstoff ein gereinigtes Salmonella typhi-Vi-Polysaccharid, ein Hepatitis A-Antigen und ein Aluminiumsalz umfaßt, wobei das Verfahren die Herstellung eines Salmonella typhi-Vi-Polysaccharids in Abwesenheit von Phenol umfaßt.

21. Verfahren gemäß Anspruch 20, worin das Polysaccharid durch Dehydratisieren mit Puffer in Abwesenheit von Phenol stabilisiert wird.

22. Verfahren gemäß Anspruch 20 oder 21, worin das Salmonella typhi-Vi-Polysaccharid mit einem inaktivierten Hepatitis A-Antigen verabreicht wird.

23. Verwendung eines Salmonella typhi-Vi-Polysaccharids, hergestellt in Abwesenheit von Phenol, in der Herstellung eines Kombinationsimpfstoffs, wobei der Impfstoff ein gereinigtes Salmonella typhi-Vi-Polysaccharid, ein Hepatitis A-Antigen und ein Aluminiumsalz umfaßt.

24. Verwendung gemäß Anspruch 23, worin der Kombinationsimpfstoff gegen Typhus und Hepatitis A ist.
